# FASCICULE DE BREVET EUROPEEN

(11) **EP 4 101 915 B1**
(45) Date de publication et mention de la délivrance du brevet: **07.02.2024**
(21) Numéro de dépôt: 22176951.6
(22) Date de dépôt: 02.06.2022
(51) Int. Cl.: C10L 3/10, F25J 3/02

(54) **INSTALLATION COMBINÉE DE SÉPARATION CRYOGÉNIQUE ET DE LIQUÉFACTION DU MÉTHANE ET DU DIOXYDE DE CARBONE COMPRIS DANS UN FLUX DE BIOGAZ**
KOMBINIERTE ANLAGE ZUR KRYOGENEN ABSCHEIDUNG UND VERFLÜSSIGUNG VON METHAN UND KOHLENDIOXID AUS EINEM BIOGASSTROM
COMBINED INSTALLATION FOR CRYOGENIC SEPARATION AND LIQUEFACTION OF METHANE AND CARBON DIOXIDE INCLUDED IN A FLOW OF BIOGAS

(30) Priorité: 09.06.2021 FR 2106082
(43) Date de publication de la demande: 14.12.2022
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: Tovar Ramos, Jorge Ernesto, Hangzhou, 310012 (CN)
(74) Mandataire: Air Liquide

(56) Documents cités:
- WO-A2-2011/135538
- US-A- 4 152 129
- US-A- 5 842 357
- YOUSEF AHMED M. ET AL: "New approach for biogas purification using cryogenic separation and distillation process for CO2 capture", ENERGY, vol. 156, 16 mai 2018 (2018-05-16), pages 328-351, XP055795448, AMSTERDAM, NL ISSN: 0360-5442, DOI: 10.1016/j.energy.2018.05.106

## Description

La présente invention est relative à une installation et à un procédé de production de méthane liquide et de dioxyde de carbone liquide à partir d'un flux de biogaz.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH₄) et du dioxyde de carbone (CO₂) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO₂, de 0 à 15% d'azote, de 0 à 5% d'oxygène et des composés traces.

Après une étape de prétraitement de ces contaminants, le biogaz peut être utilisé en l'état pour alimenter une chaudière ou une unité de cogénération, ou alors épuré pour obtenir un gaz qui répond aux spécifications pour injection dans le réseau de gaz naturel (ex: 3% de CO2 max).

Dans de nombreuses régions en Europe et dans le monde, le réseau de gaz naturel n'est pas toujours accessible à proximité des zones de production de déchets fermentescibles. De plus, s'il n'y a pas de besoin de chaleur sur le lieu de production de biogaz, en fonction du prix d'achat de l'électricité, la cogénération n'a pas toujours un rendement suffisant pour rentabiliser l'investissement important dans une unité de digestion. Il est alors intéressant dans ces 2 cas de transporter le biogaz vers un point de distribution ou de consommation. La liquéfaction du biogaz après épuration permettrait de transporter le biométhane à moindre coût. Selon la réglementation de certaines zones géographiques il est interdit de rejeter du CH4 dans l'environnement, ceci ajoute une contrainte supplémentaire et limite le choix des procédés de séparation du biogaz à des méthodes très efficaces.

Aujourd'hui, les procédés de purification du biogaz sont principalement basés sur des techniques d'absorption, de perméation ou d'adsorption. Ces systèmes nécessitent alors le rajout d'un module supplémentaire pour obtenir le biométhane sous forme liquide. De plus, dans la plupart des cas, le contenu en CO₂ dans le biogaz à l'issue de cette étape de purification est toujours trop important pour alimenter de tels systèmes de liquéfaction.

Il a été proposé un système de cryo piégeage basé sur les principes d'échangeurs réversibles. Ce système est basé sur la solidification du CO2 présent dans le biogaz sur une surface froide (piégeage), suivi d'une étape de sublimation ou liquéfaction du CO2 à l'aide d'une source chaude. Pour rendre une production continue de biométhane, il est alors nécessaire de travailler avec plusieurs échangeurs en parallèle. Leur solution permet de séparer et liquéfier le méthane et le CO2 en deux étapes distinctes, mais il n'est pas possible de récupérer le froid utilisé dans la solidification du CO2.

Partant de là un problème qui se pose est de fournir une méthode de séparation et de liquéfaction du méthane et du CO2 à partir de biogaz avec un minimum de perte de méthane et l'emploi d'un minimum d'opération.

Une solution de la présente invention est une installation combinée de séparation cryogénique et de liquéfaction du méthane et du dioxyde de carbone compris dans un flux de biogaz, comprenant :
- Un moyen M1 permettant de mélanger le biogaz 1 avec un gaz de recycle R,
- Un compresseur permettant de comprimer le mélange à la pression de distillation,
- Un échangeur E01 permettant de refroidir le mélange comprimé,
- Une colonne de distillation K01 alimentée par le mélange refroidi et permettant de produire du méthane en tête de colonne et un liquide enrichi en CO2 en fond de colonne,
- Un échangeur E02 permettant de liquéfier le méthane produit en tête de colonne,
- Un moyen M2 permettant de séparer le méthane liquéfié en deux parties : une partie « reflux » 3 et une partie « produit » 2,
- Un moyen M3 permettant de détendre et de chauffer le liquide enrichi en CO2 récupéré en fond de colonne et de récupérer le froid du liquide enrichi en CO2, et
- Un pot séparateur V01 permettant de recevoir le flux enrichi en CO2 issu du moyen M3 et de récupérer une vapeur de tête et du CO2 liquide 4,
   Avec
   - Le moyen M1 tel que le gaz de recycle R correspond à la vapeur de tête récupérée en sortie du pot séparateur V01, et
   - l'échangeur E01 et le moyen M3 étant confondus.

Selon le cas, l'installation selon l'invention peut présenter une ou plusieurs des caractéristiques ci-dessous :
▪ l'installation comprend en amont du moyen M1 des moyens de séchage et de désulfurisation du biogaz ;
▪ l'installation comprend en amont du moyen M1 un moyen C01 de compression du biogaz à la pression du gaz de recycle R.
▪ l'installation comprend en amont du moyen M1 un moyen C01E et/ou C02E de refroidissement du biogaz à la température ambiante.
▪ l'échangeur E02 est compris dans un circuit frigorifique fermé.
▪ le circuit frigorifique met en oeuvre comme fluide frigorigène du méthane.
▪ la colonne de distillation K01 comprend un chauffage en bas de colonne.

La présente invention a également pour objet un procédé combiné de séparation cryogénique et de liquéfaction du méthane et du dioxyde de carbone compris dans un flux de biogaz, mettant en oeuvre l'installation telle que définie précédemment, et comprenant :
a) Une étape de mélange du biogaz 1 avec un gaz de recycle R,
b) Une étape de compression du mélange à la pression de distillation,
c) Une étape de refroidissement du mélange comprimé dans l'échangeur E01,
d) Une étape de distillation du mélange refroidi dans la colonne de distillation K01 de manière à produire du méthane en tête de colonne et un liquide enrichi en CO2 en fond de colonne,
e) Une étape de liquéfaction du méthane produit en tête de colonne dans l'échangeur E02,
f) Une étape de séparation permettant de séparer le méthane liquéfié en deux parties : une partie « reflux » 3 et une partie « produit » 2,
g) Une étape de détente et de chauffage du liquide enrichi en CO2 récupéré en fond de colonne dans l'échangeur E01, et de récupération du froid du liquide enrichi en CO2 et
h) Une étape de séparation du flux enrichi en CO2 issu de l'échangeur E01 dans le pot séparateur V01 en CO2 liquide 4 et en vapeur de tête,

Avec le gaz de recycle R correspondant à la vapeur de tête produite à l'étape a).

Selon le cas le procédé selon l'invention peut présenter une ou plusieurs des caractéristiques ci-dessous :
▪ Le procédé comprend en amont de l'étape a) des étapes de séchage et de désu lfuration.
▪ Le procédé comprend en amont de l'étape a) une étape de compression du biogaz à la pression du gaz de recycle R.
▪ Le procédé comprend en amont de l'étape a) une étape de refroidissement du biogaz à température ambiante.
▪ Le procédé comprend en aval de l'étape h) une étape de chauffage du CO2 liquide de manière à le vaporiser.
▪ L'étape e) est réalisée par refroidissement du méthane produit au moyen d'un fluide frigorigène. Le produit en tête de la colonne est de la vapeur de méthane pure. Cette vapeur est liquéfiée dans un échangeur refroidi par un cycle frigorifique fermé, intégré thermiquement avec le procédé. Une partie du méthane liquide sort du circuit comme produit et une autre partie est utilisé comme reflux de la colonne.
▪ A l'étape b) le mélange est comprimé à une pression comprise entre 7 et 46 bar.

Le procédé selon l'invention permet de séparer et liquéfier les produits du biogaz dans une seule opération combinée distillation/liquéfaction. Les conditions opératoires des produits à l'entrée et sortie de la colonne et dans la section de recycle ont été calculées pour éviter la formation de CO2 solide.

L'intégration thermique entre les flux de la section de séparation et ceux du cycle frigorifique permet la récupération du froid utilisé dans la liquéfaction du CO2 et dans le recycle du méthane liquide. Il est possible de récupérer complètement ou en partie l'énergie utilisée dans la liquéfaction du CO2 si celui-ci n'est pas souhaité en tant que produit ou qu'il est utilisable à l'état gazeux.

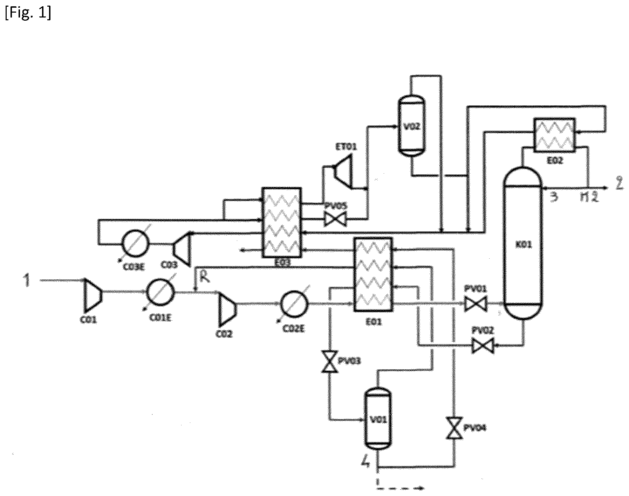

L'invention va être décrite plus en détail à l'aide de la figure 1.

Le biogaz 1 prétraité (séchage, désulfurisation) est introduit dans le procédé à pression et température atmosphériques, il est comprimé une première fois dans un compresseur C01, jusqu'à la pression du circuit de recycle (environ 8 bar). Après compression il est refroidi dans C01E à la température ambiante avec de la CW ( =Cooling Water = eau de refroidissement) ou de l'air.

Ensuite il est mélangé avec un flux de recycle R, le mélange est comprimé dans un compresseur C02, à la pression de la colonne de distillation (environ 15bar) ou plus en fonction des besoins de l'échangeur en aval E01 et il est refroidi à température ambiante dans C02E, avec de la CW ou de l'air.

De préférence C01E et C02E sont des échangeurs à tube et calandre (refroidisseur des compresseurs).

Le mélange de biogaz - flux de recycle R est envoyé dans l'échangeur E01. Le but principal de cet échangeur est de refroidir le mélange en préparation pour la distillation. Le mélange peut ensuite être détendu ou alimenté directement à la colonne où il sera utilisé comme rebouilleur.

S'il n'y a pas de source de chaleur en fond de colonne il faut injecter le mélange dans le fond pour assurer la circulation de vapeur depuis le fond. S'il y a une source de chaleur dans le fond de la colonne (rebouilleur), l'introduction du mélange est fait plus haut dans la colonne.

La colonne de distillation K01 sépare le méthane du dioxyde de carbone. L'alimentation de la colonne est le mélange biogaz + flux de recycle R. Cette alimentation agit comme rebouilleur principal ; une source complémentaire de chaleur peut être aussi utilisée (par exemple une résistance électrique, de la vapeur ou une partie du biogaz chaud en contact indirect). Le produit en tête de la colonne est du CH4 pur à l'état vapeur. Le produit de fond est un liquide riche en CO2 à environ 95%-98%.

Le méthane en tête de la colonne est liquéfié dans l'échangeur E02, contre un fluide issue d'un circuit frigorifique fermé. Une partie du méthane sort du cycle en tant que produit 2 et l'autre partie 3 (partie reflux) est utilisée comme recycle de la colonne et réinjectée en tête de colonne.

Le liquide enrichi en CO2 récupéré en fond de colonne est détendu et chauffé dans l'échangeur E01 à contrecourant du mélange biogaz - flux de recycle R.

Le flux enrichi en CO2 issu de l'échangeur E01 est envoyé dans le pot séparateur V01.

La vapeur de tête du pot V01 est réchauffée dans l'échangeur E01 et ensuite mélangée au biogaz. Elle correspond au flux nommé précédemment « flux de recycle R ».

Le liquide du fond du pot V01 est le CO2 pur 4. Celui-ci peut, en fonctions des besoins, sortir du procédé en tant que produit ou être réchauffé dans l'échangeur E01 et dans un autre échangeur E03 du circuit frigorifique pour être vaporisé complètement avant de sortir du cycle. Notons que le CO2 pur pourra alternativement être réchauffé et vaporisé dans l'échangeur E03 sans passer par l'échangeur E01.

L'échangeur E01 utilise donc comme sources de froid : le liquide enrichi en CO2 récupéré en fond de colonne, la vapeur de tête du pot V01 appelée « flux de recycle R » en sortie de l'échangeur E01, et éventuellement le CO2 pur liquide récupéré au fond du pot V01 dans le cas où sa vaporisation est souhaitée.

Le procédé nécessite un apport de puissance frigorifique pour fonctionner. Cet apport de froid est représenté dans la figure 1 par le circuit frigorifique, il est composé :
- d'un compresseur C03 avec refroidisseur C03E
- Un échangeur E03 qui refroidit le fluide comprimé à partir du fluide frigorigène recyclé et du froid récupéré du cycle de séparation.
- Une turbine ET01 et une vanne JT PV05, pour la détente du fluide frigorigène et production de froid.
- Un pot séparateur V02 pour séparer les phases vapeur et liquide du fluide frigorigène.
- Un échangeur E02 qui utilise la phase liquide du fluide frigorigène pour liquéfier le biométhane en tête de la colonne de distillation.
- Le fluide frigorigène utilisé dans le schéma est du CH4 mais il peut être remplacé par d'autres fluides comme N2, N2+H2, entre autres.

Ce cycle frigorifique peut être remplacé par d'autres sources de froid (en fonction de la quantité de biométhane liquide à produire). A titre d'exemple, mais pas exclusivement :
- A partir d'une source d'azote liquide ;
- Par un procédé type Cycle Brayton.

## Revendications

1. Installation combinée de séparation cryogénique et de liquéfaction du méthane et du dioxyde de carbone compris dans un flux de biogaz, comprenant :
- Un moyen M1 permettant de mélanger le biogaz (1) avec un gaz de recycle R,
- Un compresseur permettant de comprimer le mélange à la pression de distillation,
- Un échangeur E01 permettant de refroidir le mélange comprimé,
- Une colonne de distillation K01 alimentée par le mélange refroidi et permettant de produire du méthane en tête de colonne et un liquide enrichi en CO2 en fond de colonne,
- Un échangeur E02 permettant de liquéfier le méthane produit en tête de colonne,
- Un moyen M2 permettant de séparer le méthane liquéfié en deux parties : une partie « reflux » (3) et une partie « produit » (2),
- Un moyen M3 permettant de détendre et de chauffer le liquide enrichi en CO2 récupéré en fond de colonne et de récupérer le froid du liquide enrichi en CO2, et
- Un pot séparateur V01 permettant de recevoir le flux enrichi en CO2 issu du moyen M3 et de récupérer une vapeur de tête et du CO2 liquide (4),
Avec
- Le moyen M1 tel que le gaz de recycle R correspond à la vapeur de tête récupérée en sortie du pot séparateur V01, et
- l'échangeur E01 et le moyen M3 étant confondus.

2. Installation selon la revendication 1, **caractérisée en ce qu'**elle comprend en amont du moyen M1 des moyens de séchage et de désulfurisation du biogaz.

3. Installation selon l'une des revendications 1 ou 2, **caractérisée en ce qu'**elle comprend en amont du moyen M1 un moyen C01 de compression du biogaz à la pression du gaz de recycle R.

4. Installation selon l'une des revendications 1 à 3, **caractérisée en ce qu'**elle comprend en amont du moyen M1 un moyen C01E et/ou C02E de refroidissement du biogaz à la température ambiante.

5. Installation selon l'une des revendications 1 à 4, **caractérisée en ce que** l'échangeur E02 est compris dans un circuit frigorifique fermé.

6. Installation selon la revendication 5, **caractérisée en ce que** le circuit frigorifique met en oeuvre comme fluide frigorigène du méthane.

7. Installation selon l'une des revendications 1 à 6, **caractérisée en ce que** la colonne de distillation K01 comprend un chauffage en bas de colonne.

8. Procédé combiné de séparation cryogénique et de liquéfaction du méthane et du dioxyde de carbone compris dans un flux de biogaz, mettant en oeuvre l'installation telle que définie dans l'une des revendications 1 à 7, et comprenant :
a) Une étape de mélange du biogaz (1) avec un gaz de recycle R,
b) Une étape de compression du mélange à la pression de distillation,
c) Une étape de refroidissement du mélange comprimé dans l'échangeur E01,
d) Une étape de distillation du mélange refroidi dans la colonne de distillation K01 de manière à produire du méthane en tête de colonne et un liquide enrichi en CO2 en fond de colonne,
e) Une étape de liquéfaction du méthane produit en tête de colonne dans l'échangeur E02,
f) Une étape de séparation permettant de séparer le méthane liquéfié en deux parties : une partie « reflux » (3) et une partie « produit » (2),
g) Une étape de détente et de chauffage du liquide enrichi en CO2 récupéré en fond de colonne dans l'échangeur E01, et de récupération du froid du liquide enrichi en CO2 et
h) Une étape de séparation du flux enrichi en CO2 issu de l'échangeur E01 dans le pot séparateur V01 en CO2 liquide (4) et en vapeur de tête,
Avec le gaz de recycle R correspondant à la vapeur de tête produite à l'étape a).

9. Procédé selon la revendication 8, **caractérisé en ce qu'**il comprend en amont de l'étape a) des étapes de séchage et de désulfuration.

10. Procédé selon l'une des revendications 8 ou 9, **caractérisé en ce qu'**il comprend en amont de l'étape a) une étape de compression du biogaz à la pression du gaz de recycle R.

11. Procédé selon l'une des revendications 8 à 10, **caractérisé en ce qu'**il comprend en amont de l'étape a) une étape de refroidissement du biogaz à température ambiante.

12. Procédé selon l'une des revendications 8 à 11, **caractérisé en ce qu'**il comprend en aval de l'étape h) une étape de chauffage du CO2 liquide de manière à le vaporiser.

13. Procédé selon l'une des revendications 8 à 12, **caractérisé en ce que** l'étape e) est réalisée par refroidissement du méthane produit au moyen d'un fluide frigorigène.

14. Procédé selon l'une des revendications 8 à 13, **caractérisé en ce qu'**à l'étape b) le mélange est comprimé à une pression comprise entre 7 et 46 bar.

## Patentansprüche

1. Kombinierte Anlage zur kryogenen Trennung und Verflüssigung von Methan und Kohlendioxid in einem Biogasstrom, die Folgendes umfasst:
- ein Mittel M1, das ein Mischen des Biogases (1) mit einem Recyclegas R ermöglicht,
- einen Kompressor, der ein Komprimieren der Mischung auf den Destillationsdruck ermöglicht,
- einen Austauscher E01, der ein Kühlen der komprimierten Mischung ermöglicht,
- eine Destillationskolonne K01, die von der gekühlten Mischung gespeist wird und die Bildung von Methan am Kopf der Kolonne und einer mit CO2 angereicherten Flüssigkeit am Boden der Kolonne ermöglicht,
- einen Austauscher E02, der ein Verflüssigen des am Kopf der Kolonne gebildeten Methans ermöglicht,
- ein Mittel M2, das ein Trennen des verflüssigten Methans in zwei Teile, einen "Rückflussteil" (3) und einen "Produktteil" (2), ermöglicht,
- ein Mittel M3, das ein Entspannen und Erwärmen der am Boden der Kolonne gewonnenen, mit CO2 angereicherten Flüssigkeit und ein Rückgewinnen der Kälte der mit CO2 angereicherten Flüssigkeit ermöglicht, und
- einen Abscheidebehälter V01, der ein Aufnehmen des mit CO2 angereicherten Stroms aus dem Mittel M3 und ein Gewinnen eines Kopfdampfes und von flüssigem CO2 (4) ermöglicht,
wobei
- das Mittel M1 derart beschaffen ist, dass das Recyclegas R dem am Auslass des Abscheidebehälters V01 gewonnenen Kopfdampf entspricht, und
- der Austauscher E01 und das Mittel M3 vereinigt sind.

2. Anlage nach Anspruch 1, **dadurch gekennzeichnet, dass** sie stromaufwärts vom Mittel M1 Mittel zum Trocknen und Entschwefeln von Biogas umfasst.

3. Anlage nach einem der Ansprüche 1 oder 2, **dadurch gekennzeichnet, dass** sie stromaufwärts vom Mittel M1 ein Mittel C01 zum Komprimieren des Biogases auf den Druck des Recyclegases R umfasst.

4. Anlage nach einem der Ansprüche 1 bis 3, **dadurch gekennzeichnet, dass** sie stromaufwärts vom Mittel M1 ein Mittel C01E und/oder C02E zum Abkühlen von Biogas auf Umgebungstemperatur umfasst.

5. Anlage nach einem der Ansprüche 1 bis 4, **dadurch gekennzeichnet, dass** der Austauscher E02 in einem geschlossenen Kühlkreislauf enthalten ist.

6. Anlage nach Anspruch 5, **dadurch gekennzeichnet, dass** im Kühlkreislauf Methan als Kühlmittel verwendet wird.

7. Anlage nach einem der Ansprüche 1 bis 6, **dadurch gekennzeichnet, dass** die Destillationskolonne K01 am Boden der Kolonne eine Heizung umfasst.

8. Kombiniertes Verfahren zur kryogenen Trennung und Verflüssigung von Methan und Kohlendioxid in einem Biogasstrom, wobei die Anlage gemäß der Definition in einem der Ansprüche 1 bis 7 verwendet wird, und das Folgendes umfasst:
a) einen Schritt des Mischens des Biogases (1) mit einem Recyclegas R,
b) einen Schritt des Komprimierens der Mischung auf den Destillationsdruck,
c) einen Schritt des Kühlens der komprimierten Mischung im Austauscher E01,
d) einen Schritt des Destillierens der gekühlten Mischung in der Destillationskolonne K01, um Methan am Kopf der Kolonne und eine mit CO2 angereicherte Flüssigkeit am Boden der Kolonne zu bilden,
e) einen Schritt des Verflüssigens des am Kopf der Kolonne gebildeten Methans im Austauscher E02,
f) einen Trennschritt, der ein Trennen des verflüssigten Methans in zwei Teile, einen "Rückflussteil" (3) und einen "Produktteil" (2), ermöglicht,
g) einen Schritt des Entspannens und Erwärmens der am Boden der Kolonne gewonnenen, mit CO2 angereicherten Flüssigkeit im Austauscher E01 und des Rückgewinnens der Kälte der mit CO2 angereicherten Flüssigkeit und
h) einen Schritt des Trennens des mit CO2 angereicherten Stroms aus dem Austauscher E01 im Abscheidebehälter V01 in flüssiges CO2 (4) und Kopfdampf,
wobei das Recyclegas R dem in Schritt a) gebildeten Kopfdampf entspricht.

9. Verfahren nach Anspruch 8, **dadurch gekennzeichnet, dass** es vor Schritt a) Trocknungs- und Entschwefelungsschritte umfasst.

10. Verfahren nach einem der Ansprüche 8 oder 9, **dadurch gekennzeichnet, dass** es vor Schritt a) einen Schritt des Komprimierens des Biogases auf den Druck des Recyclegases R umfasst.

11. Verfahren nach einem der Ansprüche 8 bis 10, **dadurch gekennzeichnet, dass** es vor Schritt a) einen Schritt des Abkühlens des Biogases auf Umgebungstemperatur umfasst.

12. Verfahren nach einem der Ansprüche 8 bis 11, **dadurch gekennzeichnet, dass** es vor Schritt h) einen Schritt des Erwärmens des flüssigen CO2 umfasst, um es zu verdampfen.

13. Verfahren nach einem der Ansprüche 8 bis 12, **dadurch gekennzeichnet, dass** Schritt e) durch ein Kühlen des erzeugten Methans mit einem Kühlmittel erfolgt.

14. Verfahren nach einem der Ansprüche 8 bis 13, **dadurch gekennzeichnet, dass** in Schritt b) die Mischung auf einen Druck zwischen 7 und 46 bar komprimiert wird.

## Claims

1. Combined facility for the cryogenic separation and liquefaction of methane and carbon dioxide in a biogas stream, comprising:
- a means M1 for mixing the biogas (1) with a recycle gas R,
- a compressor for compressing the mixture to the distillation pressure,
- an exchanger E01 for cooling the compressed mixture,
- a distillation column K01 supplied with the cooled mixture and making it possible to produce methane at the top of the column and a CO₂-enriched liquid at the bottom of the column,
- an exchanger E02 for liquefying the methane produced at the top of the column,
- a means M2 for separating the liquefied methane into two portions: a "reflux" portion (3) and a "product" portion (2),
- a means M3 for expanding and heating the CO₂-enriched liquid recovered at the bottom of the column and for recovering the cold from the CO₂-enriched liquid, and
- a separator vessel V01 for receiving the CO₂-enriched stream from the means M3 and for recovering an overhead vapour and liquid CO₂ (4),
with
- the means M1 such that the recycle gas R corresponds to the overhead vapour recovered at the outlet of the separator vessel V01, and
- the exchanger E01 and the means M3 being combined.

2. Facility according to Claim 1, **characterized in that** it comprises, upstream of the means M1, means for drying and desulfurization of the biogas.

3. Facility according to either of Claims 1 and 2, **characterized in that** it comprises, upstream of the means M1, a means C01 for compressing the biogas to the pressure of the recycle gas R.

4. Facility according to one of Claims 1 to 3, **characterized in that** it comprises, upstream of the means M1, a means C01E and/or C02E for cooling the biogas to ambient temperature.

5. Facility according to one of Claims 1 to 4, **characterized in that** the exchanger E02 is within a closed refrigeration circuit.

6. Facility according to Claim 5, **characterized in that** the refrigeration circuit uses methane as refrigerant fluid.

7. Facility according to one of Claims 1 to 6, **characterized in that** the distillation column K01 comprises heating at the bottom of the column.

8. Combined process for the cryogenic separation and liquefaction of methane and carbon dioxide within a biogas stream, using the facility as defined in one of Claims 1 to 7, and comprising:
a) a step of mixing the biogas (1) with a recycle gas R,
b) a step of compressing the mixture to the distillation pressure,
c) a step of cooling the compressed mixture in the exchanger E01,
d) a step of distilling the cooled mixture in the distillation column K01 so as to produce methane at the top of the column and a CO₂-enriched liquid at the bottom of the column,
e) a step of liquefying the methane produced at the top of the column in the exchanger E02,
f) a separation step for separating the liquefied methane into two portions: a "reflux" portion (3) and a "product" portion (2),
g) a step of expanding and heating the CO₂-enriched liquid recovered at the bottom of the column in the exchanger E01, and of recovering the cold from the CO₂-enriched liquid, and
h) a step of separating the CO₂-enriched stream resulting from the exchanger E01 in the separator vessel V01 into liquid CO₂ (4) and overhead vapour,
with the recycle gas R corresponding to the overhead vapour produced in step a).

9. Process according to Claim 8, **characterized in that** it comprises, upstream of step a), steps of drying and of desulfurization.

10. Process according to either of Claims 8 and 9, **characterized in that** it comprises, upstream of step a), a step of compressing the biogas to the pressure of the recycle gas R.

11. Process according to one of Claims 8 to 10, **characterized in that** it comprises, upstream of step a), a step of cooling the biogas to ambient temperature.

12. Process according to one of Claims 8 to 11, **characterized in that** it comprises, downstream of step h) , a step of heating the liquid CO₂ so as to vaporize it.

13. Process according to one of Claims 8 to 12, **characterized in that** step e) is performed by cooling the methane produced by means of a refrigerant fluid.

14. Process according to one of Claims 8 to 13, **characterized in that**, in step b), the mixture is compressed to a pressure of between 7 and 46 bar.
